# DEMANDE DE BREVET EUROPEEN

(11) **EP 1 477 188 A1**
(43) Date de publication de la demande: **17.11.2004**
(21) Numéro de dépôt: 04405288.4
(22) Date de dépôt: 07.05.2004
(51) Int. Cl.: A61L 2/14, A61F 2/30, A61C 8/00, C23C 4/02, A61L 2/12, A61L 2/10

(54) **Procédé de nettoyage et de décontamination bactérienne de pièces mécaniques à usage médical, et dispositif pour la mise en oeuvre de ce procédé**

(30) Priorité: 12.05.2003 FR 0305666
(71) Demandeur: Terolab Services Management SA, 1018 Lausanne (CH)
(72) Inventeur: Murano, Donato, 94480 Ablon-sur-Seine (FR)
(74) Mandataire: Nithardt, Roland

(57) **Abrégé**

La présente invention concerne un procédé et un dispositif de nettoyage et de décontamination de pièces mécaniques à usage médical, notamment d'implants dentaires ou orthopédiques. Ce dispositif (10) comporte des moyens de traitement pour soumettre les pièces à un traitement au plasma sous atmosphère contrôlée assisté par micro-ondes ou ondes de radio fréquences. Ces moyens de traitement comprennent un générateur de plasma (12) dit froid et un générateur (13) de micro-ondes ou d'ondes de radio fréquences. Ces appareils sont logés dans une enceinte (11) dans laquelle sont placées des pièces à traiter (16) disposées sur un plateau tournant (18).

## Description

La présente invention concerne un procédé de nettoyage et de décontamination de pièces mécaniques à usage médical, notamment d'implants dentaires ou orthopédiques, dans lequel l'on soumet les pièces à un traitement au plasma dit plasma froid à décharge, sous atmosphère contrôlée.

Elle concerne également un dispositif de nettoyage et de décontamination de pièces mécaniques à usage médical, notamment d'implants dentaires ou orthopédiques, pour la mise en oeuvre de ce procédé, comportant des moyens de traitement pour soumettre les pièces à un traitement au plasma dit plasma froid à décharge.

La métallisation des prothèses, notamment des prothèses orthopédiques, par projection thermique est utilisée depuis de nombreuses années et donne d'excellents résultats. La mise en oeuvre de ce procédé implique une préparation minutieuse de la surface, notamment un dégraissage au moyen de bains lessiviels ou de solvants et un traitement tendant à favoriser l'adhérence du revêtement qui s'opère habituellement selon des méthodes bien connues de sablage ou de grenaillage au moyen de grains de matériaux abrasifs ou de corindonnage au moyen de grains d'alumine. Ces techniques ont pour effet de générer des rugosités à la surface des pièces, ce qui est souvent recherché parce qu'elles permettent d'augmenter la valeur géométrique de cette surface et favorise l'adhérence des revêtements.

Cependant ces techniques présentent l'inconvénient d'incruster des particules de matériaux abrasifs ou d'alumine dans les pièces traitées, de sorte qu'un nettoyage postérieur au sablage ou au grenaillage est requis. Ce nettoyage s'effectue souvent par lessivage ou trempage ou par aspersion. Dans certains cas, le nettoyage s'effectue sous la forme d'un traitement chimique au moyen d'un bain. On connaît de nombreux procédés qui utilisent des bains de traitement pour nettoyer des pièces de prothèses après une opération de sablage ou de grenaillage.

Le brevet américain US 6,183,255 décrit par exemple un traitement chimique au moyen d'hydroxyde de sodium seul ou suivi d'un traitement par une solution aqueuse d'acide fluorhydrique ou d'acide nitrique.

La publication WO 02/24243 A1 décrit un procédé de traitement des implants en titane au moyen de trois acides qui sont l'acide fluorhydrique, l'acide sulfurique et l'acide chlorhydrique suivi d'un traitement au plasma.

Le brevet américain US 4,075,040 décrit un procédé de traitement de surface de pièces en titane ou en alliages de titane au moyen d'un bain d'acide contenant des fluorures.

Le brevet US 5,476,552 décrit un procédé de traitement de la surface de pièces métalliques comprenant un sablage suivi d'un traitement chimique par une solution acide contenant de l'alcool isopropylique, de l'alcool éthylique, de l'alcool buthylique, du chromate de zinc et de l'acide phosphorique.

Le brevet US 4,292,090 décrit un procédé de traitement de la surface de pièces en titane par immersion dans une solution alcaline contenant des ions de calcium. Ce procédé est destiné à éliminer l'oxyde de titane.

Ces opérations sont toutes fastidieuses et coûteuses et ne répondent pas de manière satisfaisante et fiable aux exigences en matière de préparation de la surface de prothèses orthopédiques, par exemple, en vue d'un traitement postérieur de métallisation par projection thermique. En outre, les bains doivent être régulièrement contrôlés et les effluents doivent être recyclés. De plus on risque de modifier les caractéristiques de la surface des pièces par absorption d'humidité ou d'agression chimique. Enfin, certains contaminants ne sont pas systématiquement détachés de la surface des prothèses. D'un point de vue économique, la pluralité des interventions constitue un handicap en raison du coût de ces interventions et des équipements nécessaires.

C'est pourquoi de nouvelles techniques ont été développées qui sont destinées à permettre d'éviter les inconvénients des procédés cités ci-dessus.

Parmi ces techniques, on peut mentionner le procédé de préparation de la surface d'implants décrit par la publication européenne EP 0 606 566 A. Ce procédé de traitement de la surface s'effectue au moyen d'un plasma à décharge de gaz, les implants, préalablement nettoyés, étant introduits dans une chambre à vide. Le traitement au plasma s'effectue en deux phases. La première consiste à effectuer un traitement au plasma sous atmosphère neutre et la seconde consiste à effectuer un traitement au plasma sous atmosphère oxydante.

La publication européenne EP 0 864 374 A décrit un procédé de traitement de la surface de pièces de prothèses dentaires, ce procédé consistant à effectuer plusieurs phases de traitements au plasma en vue d'améliorer l'adhésion d'une couche de matière synthétique.

La demande internationale de brevet publiée sous le numéro WO 97/26026 A décrit une méthode permettant d'obtenir, par un traitement au plasma avec un gaz réactif, une surface poreuse pour des implants médicaux, notamment en titane.

La demande de brevet allemand publiée sous le numéro DE 196 44 153 A1 décrit un procédé de traitement au plasma de pièces en vue de leur nettoyage, dégraissage et activation. Ce traitement s'effectue en plusieurs phases successives, chacune de ces phases ayant une action différente.

Le brevet américain US 5 188 800 décrit un procédé de nettoyage et de stérilisation d'implants dentaires par un traitement au plasma. Ce plasma est généré de manière classique par l'ionisation du gaz contenu dans la chambre de traitement par un arc électrique entre une cathode et une anode.

Le brevet américain US 6 588 621 B1 décrit un procédé de décontamination d'implants médicaux au moyen d'oxygène atomique généré par un plasma.

La demande de brevet européen EP 0 523 372 A décrit un procédé de projection thermique destiné à appliquer des composants sur un implant médical.

Aucun de ces documents ne mentionne en particulier l'utilisation de micro-ondes pour assister le plasma.

Le but de l'invention est de proposer un procédé de nettoyage efficace, fiable économique, modulable et rapide permettant de préparer la surface de pièces mécaniques, en particulier de prothèses dentaires, orthopédiques ou similaires, de les nettoyer, de les dégraisser et de les décontaminer antérieurement à un traitement de surface ultérieur, en particulier une opération de revêtement de surface par projection thermique.

Ce but est atteint par le procédé selon l'invention tel que défini en préambule et caractérisé en ce que ledit plasma froid est assisté par une source de hautes fréquences, en particulier un générateur de micro-ondes.

Selon un mode de réalisation avantageux, l'on effectue ledit traitement des pièces au plasma froid dans une enceinte contenant une atmosphère contrôlée, les pièces étant déplacées pour exposer toutes leurs faces au plasma.

D'une manière préférentielle, l'on procède audit traitement des pièces au plasma froid et l'on effectue immédiatement un revêtement de surface par projection thermique sur les pièces traitées.

Selon un premier mode de mise en oeuvre, l'on effectue ledit traitement des pièces au plasma froid et ledit revêtement de surface par projection thermique dans des enceintes différentes.

Selon un deuxième mode de mise en oeuvre, l'on effectue ledit traitement des pièces au plasma froid et ledit revêtement de surface par projection thermique dans la même enceinte.

Selon un troisième mode de mise en oeuvre, l'on effectue ledit traitement des pièces au plasma froid et ledit revêtement de surface par projection thermique dans deux chambres contiguës de la même enceinte.

Dans ce cas, l'on déplace de préférence les pièces successivement à travers une première chambre de traitement au plasma froid et à travers une deuxième chambre de revêtement de surface par projection thermique.

Ce but est également atteint par le dispositif selon l'invention tel que défini en préambule et caractérisé en ce qu'il comporte des moyens d'assistance comprenant une source de hautes fréquences, constituée d'un générateur de micro-ondes.

Lesdits moyens de traitement au plasma froid sont de préférence disposés dans une enceinte associée à des moyens pour générer le vide ou une atmosphère contrôlée.

Pour permettre d'effectuer un traitement uniforme des pièces, ladite enceinte contient des moyens de support desdites pièces. Ces moyens de support des pièces sont avantageusement mobiles.

Selon un mode de réalisation avantageux, ladite enceinte comporte une première chambre de traitement au plasma froid et une deuxième chambre de revêtement de surface par projection thermique adjacente à la première chambre de traitement au plasma froid.

La présente invention et ses avantages seront mieux compris à la lecture de la description détaillée d'une mise en oeuvre préférée du procédé de l'invention, en référence aux dessins annexés donnés à titre indicatif et non limitatif, dans lesquels:
- la figure 1 est une vue schématique représentant une première forme de réalisation du dispositif selon l'invention, et
- la figure 2 représente une vue schématique d'une deuxième forme de réalisation du dispositif selon l'invention.

Au cours d'une phase préliminaire, les prothèses orthopédiques sont généralement revêtues, au moins en partie, d'au moins une couche métallique ou d'une couche de céramique par projection au plasma. Dans le cas d'un revêtement métallique, cette couche est par exemple constituée de titane, de chrome, de cobalt ou de leurs alliages ou d'une combinaison de plusieurs composés métalliques. Dans le cas d'une couche de céramique, elle est par exemple constituée de zircone, de carbonate de calcium, d'alumine ou d'hydroxyapatite ou d'une combinaison contenant certains de ces composants. Après ce revêtement, les prothèses sont démasquées, c'est-à-dire que l'on enlève, le cas échéant, les éléments protecteurs, caches métalliques, ruban adhésif, pièces surmoulées en matière plastique ou en matériaux composites. Ensuite elles sont parachevées par ébavurage, par enlèvement des surplus de revêtement ou par un éventuel enlèvement des traces d'adhésif au moyen de solvants tels que de l'acétone, de l'alcool dénaturé ou similaire. Un soufflage à l'air comprimé propre et sec a pour objectif d'éliminer les poussières et les résidus des matériaux d'apport.

L'ensemble de ces démarches génère une contamination qui doit impérativement être éliminée au cours d'une phase de nettoyage et de décontamination devant survenir avant la préparation finale des implants orthopédiques ou dentaires, par exemple avant l'emballage final et la stérilisation, ou au cours d'une phase intermédiaire, notamment dans le cas où l'on applique un revêtement bi-couche tel que par exemple du titane et de l'hydroxyapatite. Cette phase de nettoyage peut aussi être pratiquée après projection d'une sous-couche métallique, par exemple en titane, ou en céramique, notamment en alumine, et avant la projection de la couche finale, par exemple en hydroxyapatite ou en carbonate de calcium. Pour cette décontamination, l'utilisation de solutions aqueuses, qui intervient habituellement au cours de cette phase de nettoyage, notamment pour assurer une décontamination bactérienne indispensable pour tous les produits à usage médical, présente de nombreux inconvénients et est supprimée dans le cadre de la présente invention.

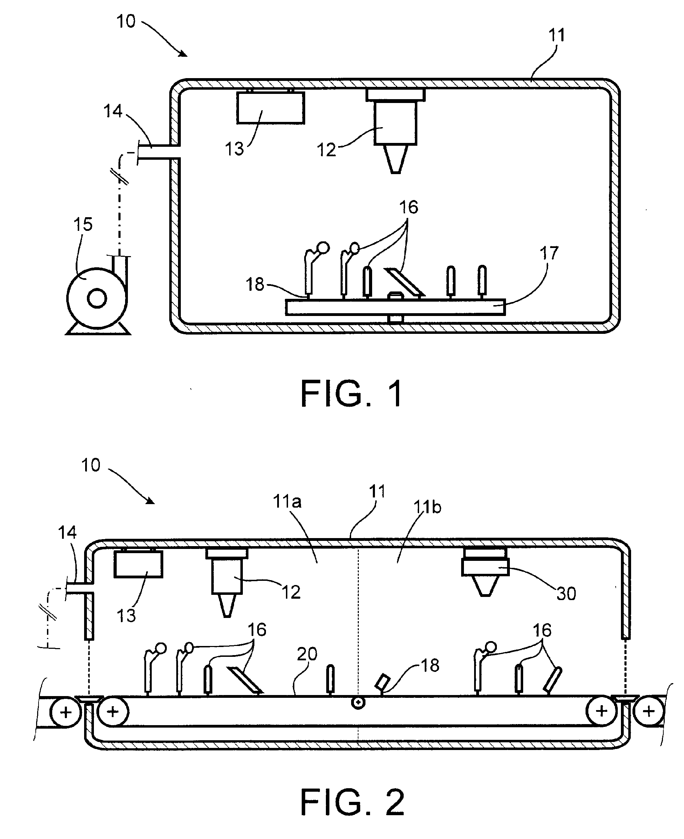

Le nettoyage et la décontamination sont effectués par un traitement au plasma à décharge, le générateur de plasma étant de préférence assisté par micro-ondes ou par ondes de radio fréquences. Comme le montre la figure 1, le dispositif 10 comporte essentiellement une enceinte 11 constituée d'une cavité résonnante et contenant un générateur de plasma 12 et un générateur de micro-ondes ou d'ondes de radio fréquences 13. L'enceinte est de préférence réalisée en aluminium ou en tout autre matériau approprié et comporte des moyens de raccordement 14 à une pompe à vide 15 ou à un dispositif agencé pour créer une atmosphère contrôlée à l'intérieur de cette enceinte 11.

Les pièces 16 à nettoyer et à décontaminer, représentées schématiquement, sont disposées sur un plateau 17 qui peut être un plateau tournant ou fixe. Les pièces 16 peuvent être montées sur des supports tournants ou fixes 18 selon leur taille et selon la complexité de leur forme. Pour des pièces de faible dimension telles que par exemple des implants dentaires, elles peuvent être disposées en vrac.

La figure 2 illustre une autre forme de réalisation du dispositif 10 qui comporte essentiellement une enceinte 11 composée d'une chambre 11a de nettoyage et de décontamination par traitement au plasma et d'une chambre 11 b de revêtement par projection thermique, qui sont juxtaposées de telle manière que les pièces à traiter passent directement de l'une à l'autre. Comme précédemment, un générateur de plasma 12 et un générateur de micro-ondes ou d'ondes de radio fréquences 13 sont installés dans l'enceinte 11, plus précisément dans la chambre 11 a. L'enceinte 11 est de préférence réalisée en aluminium ou en tout autre matériau approprié et comporte des moyens de raccordement 14 à une pompe à vide (non représentée) ou à un dispositif agencé pour créer une atmosphère contrôlée à l'intérieur de ladite enceinte. La chambre adjacente 11 b contient un dispositif de revêtement par projection thermique 30. Les pièces 16 sont par exemple transportées sur un tapis transporteur 20 et montées sur des supports 18, éventuellement tournants, de manière telle qu'elles soient exposées sur toutes leurs faces au plasma de traitement. Les pièces peuvent également être déposées en vrac sur un tapis vibrant ou sur tout autre élément de support ou de transport.

Le plasma est généré par l'excitation et l'ionisation du gaz par l'énergie procurée par la source de hautes fréquences. La décharge est du type magnétron lorsqu'elle est accompagnée d'un champ magnétique intense, notamment dans un champ électromagnétique micro-ondes, ce qui conduit à la résonance cyclotronique électronique (ECR). L'utilisation des micro-ondes à la place des ondes de radio fréquences apporte une meilleure réactivité de par la forte densité des électrons.

Etant donné que les opérations de décontamination sont obligatoirement effectuées dans une atmosphère contrôlée, l'enceinte 11 se trouve dans un local approprié, appelé salle propre, correspondant aux exigences du traitement. Le plasma utilisé pour l'opération de décontamination est de préférence du type dit «plasma froid» sous vide capable de préserver la pièce, c'est-à-dire le substrat et son revêtement. La biocharge doit être comprise entre 100 et 0 UFC.

A titre d'exemple, le traitement d'une pièce métallique ayant un revêtement métallique ou en céramique s'effectue de préférence dans une enceinte 11 équipée d'un plateau mobile, le générateur de plasma ayant une puissance de l'ordre de 880 watts, le gaz primaire étant l'oxygène et le gaz secondaire étant l'argon. La durée du cycle de traitement est de quinze minutes. Pour une pièce métallique comportant un polymère, par exemple un corps gras, la puissance du générateur de plasma est de préférence de l'ordre de 600 watts, le gaz primaire est l'oxygène, le gaz secondaire est l'argon ou un mélange d'oxygène et d'argon. La pièce est avantageusement déposée sur un plateau mobile.

Les résultats sont probants et tous les contaminants organiques ou inorganiques sont éliminés sur des revêtements poreux, microporeux ou homogènes sans qu'une quelconque modification préjudiciable au traitement postérieur de revêtement par projection thermique soit constatée.

La forme de réalisation décrite n'est pas limitative par rapport à l'invention dans sa généralité. D'autres générateurs de plasma peuvent être utilisés et notamment des générateurs mobiles pouvant être manipulés de façon flexible à la manière d'un stylo, les pièces étant maintenues immobiles ou montées sur des supports mobiles.

L'utilisation des micro-ondes produit plus d'énergie que les systèmes à courant alternatif, les systèmes à ondes audio et les systèmes à ondes de radio fréquences. Les surfaces ne subissent aucune modification et les pièces n'entrent pas en jeu pour la génération du plasma puisqu'elles ne sont pas utilisées comme des électrodes. Tous les types de substrats peuvent être traités. La durée du cycle de traitement et les paramètres peuvent être ajustés librement en fonction du résultat visé.

## Revendications

1. Procédé de nettoyage et de décontamination de pièces mécaniques à usage médical, notamment d'implants dentaires ou orthopédiques, dans lequel l'on soumet les pièces à un traitement au plasma dit plasma froid à décharge alimenté en continu ou en radio fréquences, sous atmosphère contrôlée, **caractérisé en ce que** ledit plasma froid est assisté par une source de hautes fréquences, en particulier un générateur de micro-ondes.

2. Procédé selon la revendication 1, **caractérisé en ce que** l'on effectue ledit traitement des pièces au plasma froid dans une enceinte contenant une atmosphère contrôlée, les pièces étant déplacées pour exposer toutes leurs faces au plasma froid.

3. Procédé selon la revendication 1, **caractérisé en ce que** l'on procède audit traitement des pièces au plasma froid et **en ce que** l'on effectue, immédiatement après ce traitement, un revêtement de surface par projection thermique sur les pièces traitées.

4. Procédé selon la revendication 3, **caractérisé en ce que** l'on effectue ledit traitement des pièces au plasma froid et ledit revêtement de surface par projection thermique dans des enceintes différentes.

5. Procédé selon la revendication 3, **caractérisé en ce que** l'on effectue ledit traitement des pièces au plasma froid et ledit revêtement de surface par projection thermique dans la même enceinte.

6. Procédé selon la revendication 3, **caractérisé en ce que** l'on effectue ledit traitement des pièces au plasma froid et ledit revêtement de surface par projection thermique dans deux chambres contiguës de la même enceinte.

7. Procédé selon la revendication 6, **caractérisé en ce que** l'on déplace les pièces successivement à travers une première chambre de traitement au plasma froid et à travers une deuxième chambre de revêtement de surface par projection thermique.

8. Dispositif de nettoyage et de décontamination de pièces mécaniques à usage médical, notamment d'implants dentaires ou orthopédiques, pour la mise en oeuvre du procédé selon la revendication 1, comportant des moyens de traitement (12) pour soumettre les pièces (16) à un traitement au plasma dit plasma froid à décharge, **caractérisé en ce qu'**il comporte des moyens d'assistance comprenant une source de hautes fréquences, constituée d'un générateur de micro-ondes (13).

9. Dispositif selon la revendication 8, **caractérisé en ce que** lesdits moyens de traitement sont disposés dans une enceinte (11) associée à des moyens (15) pour générer le vide ou une atmosphère contrôlée.

10. Dispositif selon la revendication 9, **caractérisé en ce que** ladite enceinte (11) contient des moyens de support (17, 18) desdites pièces (16).

11. Dispositif selon la revendication 10, **caractérisé en ce que** lesdits moyens de support (17, 18) des pièces (16) sont mobiles.

12. Dispositif selon la revendication 10, **caractérisé en ce que** ladite enceinte (11) comporte une première chambre (11a) de traitement au plasma à froid et une deuxième chambre (11 b) de revêtement de surface par projection thermique adjacente à la première chambre de traitement au plasma (11a).
